(19) Europäisches Patentamt
European Patent Office
Office européen des brevets

(11) Veröffentlichungsnummer: **0 168 761**
**B1**

(12) # EUROPÄISCHE PATENTSCHRIFT

(45) Veröffentlichungstag der Patentschrift: **01.04.87**

(51) Int. Cl.[4]: **C 07 C 47/21**, C 07 C 45/60, C 07 C 45/67

(21) Anmeldenummer: **85108551.4**

(22) Anmeldetag: **10.07.85**

(54) **Verfahren zur Herstellung von 2-Methyl-2-alkenalen aus Dihydropyranen.**

(30) Priorität: **19.07.84 DE 3426544**

(43) Veröffentlichungstag der Anmeldung: **22.01.86 Patentblatt 86/4**

(45) Bekanntmachung des Hinweises auf die Patenterteilung: **01.04.87 Patentblatt 87/14**

(84) Benannte Vertragsstaaten: **BE CH DE FR GB IT LI NL**

(56) Entgegenhaltungen:
**DE - A - 2 620 967**
**DE - A - 2 621 224**
**US - A - 4 433 174**

**JUSTUS LIEBIGS ANNALEN DER CHEMIE, Band 494, 1932, Verlag Chemie, G.M.B.H., Berlin. F. G. FISCHER et al.: "Die Synthese der Dehydro-geraniumsäure und anderer Verbindungen aus 2-Methyl-buten-(2)-al-(4)" Seiten 263-284**

(73) Patentinhaber: **BASF Aktiengesellschaft, Carl-Bosch-Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Fischer, Rolf, Dr., Bergstrasse 98, D-6900 Heidelberg (DE)**
Erfinder: **Merger, Franz, Dr., Max-Slevogt-Strasse 25, D-6710 Frankenthal (DE)**
Erfinder: **Mross, Wolf Dieter, Dr., Anselm-Feuerbach-Strasse 21, D-6710 Frankenthal (DE)**

**EP 0 168 761 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Methyl-2-alkenalen durch Spaltung von Dihydropyranen an Zeolithen, Aluminosilikaten und/oder Aluminophosphaten.

Es ist bekannt, dass man 4-Acetoxy-2-formyl-1-buten in 4-Acetoxy-2-formyl-2-buten (4-Acetoxytiglinaldehyd) umwandeln kann. Bei diesem in der DE-OS 2 621 224 beschriebenen Verfahren leitet man bei höherer Temperatur Wasserstoff durch Lösungen von 4-Acetoxy-2-formyl-1-buten, in denen ein mit Schwefel dotierter Palladium-Trägerkatalysator suspendiert ist. Man isoliert Gemische aus 4-Acetoxy-2-formyl-2-buten und 4-Acetoxy-2-formylbutan im Verhältnis 1:4. Es ist ferner bekannt, dass sich α-Ethylacrolein durch Behandlung mit Calciumchlorid und anschliessende Destillation zu Tiglinaldehyd (trans-2-Methyl-2-butenal) umlagern lässt (Liebigs Ann. *494*, 273 (1932)). Um 2-Alkylacrolein in die entsprechende 2-Methyl-2-alkenale umzuwandeln; kann man auch so verfahren, dass man zunächst die jeweiligen 2-Alkylacroleindialkylhydrazone herstellt, diese mit katalytischen Mengen starker Säuren in die Dialkylhydrazone der entsprechenden 2-Methyl-2-alkenale umlagert und dann aus den Hydrazonen die 2-Methyl-2-alkenale durch Hydrolyse freisetzt (Zh. Org. Khim. *5*, 1183 (1969)).

Die Isomerisierung an Palladium-Katalysatoren hat den Nachteil, dass beträchtliche Mengen des Ausgangsprodukts zu dem nicht verwendbaren 4-Acetoxy-2-formyl-butan hydriert werden. Die Umwandlung von α-Ethylacrolein in Tiglinaldehyd in Gegenwart von Calciumchlorid liess sich beim Nacharbeiten der Literaturangaben nicht bestätigen. Die über die Dialkylhydrazone verlaufende Isomerisierung hat den Nachteil, dass sie drei Reaktionsschritte erfordert. Ausserdem fällt das eingesetzte Dialkylhydrazin im letzten Reaktionsschritt als Salz an und muss mit Alkali unter Neutralsalzbildung freigesetzt werden.

Da 2-Methyl-2-alkenale gesuchte Zwischenprodukte darstellen, und z.B. Tiglinaldehyd als wichtiges Vorprodukt für die Herstellung von Farbstoffen, Pharmazeutika und Pflanzenschutzmitteln benötigt wird, war nach einem Verfahren zu suchen, das es gestattet, 2-Methyl-2-alkenale auf vorteilhaftere Weise herzustellen.

Es wurde nun gefunden, dass man 2-Methyl-2-alkenale der Formel

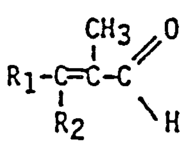

I,

in der $R_1$ und $R_2$ Wasserstoffatome, Alkylreste mit 1 bis 18 Kohlenstoffatomen oder aromatische Reste bedeuten, besonders vorteilhaft dadurch erhält, dass man Dihydropyrane der Formel

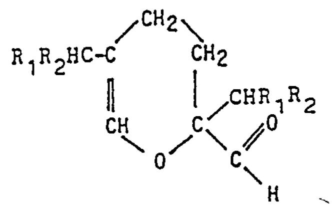

II,

in der $R_1$ und $R_2$ die obengenannte Bedeutung haben, in Gegenwart von Zeolithen, Aluminosilikaten und/oder Aluminophosphaten erhitzt und aus den dabei entstehenden Gemischen die 2-Methyl-2-alkenale abtrennt.

Nach dem neuen Verfahren erhitzt man die Dihydropyrane in Gegenwart der genannten Katalysatoren. Man arbeitet in der Gasphase oder in der Flüssigphase. In der Gasphase wählt man Temperaturen von 150 bis 450° C, vorzugsweise 300 bis 400° C. In der Flüssigphase betragen die Temperaturen 30 bis 300° C. Bevorzugt arbeitet man in der Gasphase bei einer Belastung (WHSV) von 0,1 bis 20 $h^{-1}$, bevorzugt 0,5 bis 5 $h^{-1}$ (g Dihydropyran/g Katalysator und Stunde).

Das Verfahren kann chargenweise oder kontinuierlich, drucklos oder unter Druck in einem Rohrreaktor, Rührkessel oder Wirbelbettreaktor durchgeführt werden. Man kann die Reaktion in Gegenwart von Gasen, wie Wasserstoff, Stickstoff und Wasserdampf durchführen, womit man die Produktzusammensetzung und Standzeit des Katalysators beeinflussen kann. Insbesondere durch Wasserdampfzusatz lässt sich die Desaktivierung des Katalysators zurückdrängen.

Bei dem erfindungsgemässen Verfahren tritt eine Spaltung der Dihydropyrane ein, bei der neben den 2-Methyl-2-alkenalen der Formel I die 2-Alkyl(Aryl)acroleine der Formel

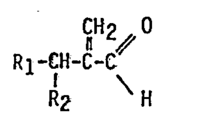

III,

entstehen. Die Spaltung der Dihydropyrane lässt sich z.B. für die Reaktion von 2,5-Diethyl-2-formyl-2,3-dihydro-4H-pyran zu Tiglinaldehyd (trans-2-Methyl-2-butenal) und Ethylacrolein durch die folgenden Formeln wiedergeben:

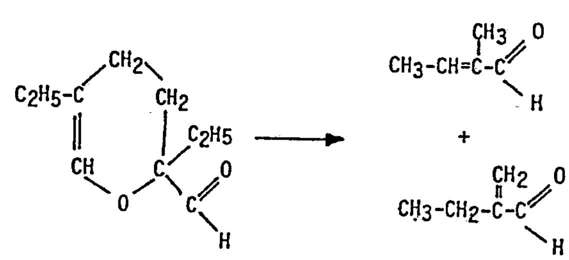

Das Ergebnis des erfindungsgemässen Verfahrens ist überraschend, da bei der thermischen Spaltung der Dihydropyrane der Formel II nur die Verbindungen der Formel III erhalten werden (s. Vergleichsbeispiel).

Die Ausgangsstoffe der Formel II enthalten als Reste $R_1$ und $R_2$ Wasserstoffatome, Alkylreste mit 1 bis 18 Kohlenstoffatomen und aromatische Reste. Als Alkylreste kommen z.B. Methyl-, Ethyl-, n-Propyl-, i-Propyl-, n-Butyl-, i-Butyl-, tert.-Butyl-, Pentyl-, Decyl- und Dodecylreste in Betracht. Als aromatische Reste kommen z.B. Phenylreste in Frage, die gegebenenfalls noch durch andere Reste, wie Alkyl- oder Alkoxygruppen oder durch Halogenatome substituiert sein können. Beispielsweise seien die folgenden Dihydropyrane der Formel II genannt: 2,5-Diethyl-2-formyl-2,3-dihydro-4H-pyran, 2,5-Dipropyl-2-formyl-2,3-dihydro-4H-pyran, 2,5-Di-n-octyl-2-formyl-2,3-dihydro-4H-pyran, 2,5-Dibenzyl-2-formyl-2,3-dihydro-4H-pyran.

Die Dihydropyrane der Formel II lassen sich z.B. durch Erhitzen von 2-Alkyl(Aryl)acroleinen der Formel III auf 140 bis 170° C, mit oder ohne Lösungsmittel, herstellen (Houben-Weyl, Methoden der organischen Chemie, 4. Auflage, Band VII, Teil 1, Seite 130).

Da beim erfindungsgemässen Erhitzen der Dihydropyrane ein Gemisch aus den 2-Methyl-2-alkenalen der Formel I und den 2-Alkyl(Aryl)acroleinen der Formel III entsteht, wird das gewünschte 2-Methyl-2-alkenal aus dem Gemisch abgetrennt. Man trennt es zweckmässigerweise durch fraktionierte Destillation ab. Die durch diese Abtrennung erhältlichen 2-Alkyl(Aryl)acroleine der Formel III werden vorteilhaft für die oben erwähnte Herstellung der Dihydropyrane der Formel II verwendet.

Als Katalysatoren für die Spaltung der Dihydropyrane werden Zeolithe eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluss von Kationen in den Kristall z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt. Als Katalysatoren sind auch kristalline Verbindungen mit Zeolithstruktur sehr geeignet, bei denen dreiwertige Elemente wie B, Ga, Fe, Cr anstelle des Aluminiums oder vierwertige Elemente wie Ge anstelle des Siliciums in das Zeolithgitter eingebaut sind.

Vorzugsweise werden als Katalysatoren Zeolithe des Pentasiltyps eingesetzt. Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemische aus Alumino-, Boro-, Gallium- und Eisengermanatzeolithe oder deren Gemische. Von diesen sind die Alumino-, Boro- und Eisensilikatzeolithe des Pentasiltyps bevorzugt.

Der Aluminosilikatzeolith wird aus einer Aluminiumverbindung, vorzugsweise $Al(OH)_3$ oder $Al_2(SO_4)_3$, und einer Siliciumkomponente, vorzugsweise hochdispersem Siliciumdioxid in wässeriger Aminlösung, insbesondere in 1,6-Hexandiamin- oder 1,3-Propandiamin- oder Triethylentriamin-Lösung mit und ohne Alkali- oder Erdalkalizusätze bei 100 bis 200° C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40 000. Man kann derartige Aluminosilikatzeolithe auch in etherischem Medium, wie Diethylenglykoldimethylether, in alkoholischem Medium, wie Methanol bzw. 1,4-Butanol oder in Wasser synthetisieren.

Der Borosilikatzeolith wird z.B. bei 90 bis 200° C unter autogenem Druck synthetisiert, indem man eine Borverbindung, z.B. $H_3BO_3$, mit einer Siliciumverbindung, vorzugsweise hochdispersem Siliciumdioxid in wässeriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Man kann solche Borosilikatzeolithe auch dadurch herstellen, dass man die Reaktion in etherischer Lösung, z.B. Diethylenglykoldimethylether, oder in alkoholischer Lösung, z.B. in 1,6-Hexandiol, durchführt.

Den Eisensilikatzeolith kann man aus einer Eisenverbindung, vorzugsweise $Fe_2(SO_4)_3$ und einer Siliciumverbindung, vorzugsweise hochdispersem Siliciumdioxid in wässeriger Aminlösung, insbesondere in 1,6-Hexandiamin, mit und ohne Alkali- oder Erdalkalizusatz bei 100 bis 200° C unter autogenem Druck herstellen.

Die so hergestellten Alumino-, Boro- und Eisensilikatzeolithe können nach ihrer Isolierung, Trocknung bei 100 bis 160° C, vorzugsweise 110° C, und Calcination bei 450 bis 550° C, vorzugsweise 500° C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge bei 110° C/16 h getrocknet und bei 500° C/16 g calciniert. Besonders vorteilhafte Katalysatoren werden dadurch erhalten, dass man isolierte Alumino- bzw. Borosilikatzeolithe direkt nach der Trocknung verformt und erstmals nach der Verformung einer Calcination unterwirft. Die hergestellten Alumino-, Boro- und Eisensilikatzeolithe können aber auch in reiner Form, ohne Binder, als Stränge oder Tabletten eingesetzt werden.

Es sind auch Aluminosilikatzeolithe des Y-Typs geeignet, die aus Kieselsol (29% $SiO_2$) und Natriumaluminat in wässerigem Medium hergestellt werden. Diese Aluminosilikatzeolithe können vor ihrem Einsatz ebenfalls mit Bindern verformt werden.

Auch Zeolithe des Mordenit-Typs und des X-Typs sind verwendbar.

Liegt der Zeolith nach seiner Synthese nicht in der katalytisch besonders aktiven, aciden H-Form,

sondern z.B. in der Na-Form vor, so kann diese durch Behandlung mit Säuren vollkommen oder partiell in die gewünschte H-Form überführt werden. Auch kann der Austausch mit Ammoniumsalzen durchgeführt werden.

Zur Erhöhung der Selektivität, der Standzeit und der Anzahl an Regenerierungen kann es zweckmässig sein, unterschiedliche Modifizierungen an den zeolithischen Katalysatoren vorzunehmen. Man modifiziert die Katalysatoren z.B. dadurch, dass man bei den unverformten oder verformten Zeolithen mit Alkalimetallen, wie Na – sofern nicht schon von der Synthese her die Alkali-Form des Zeolithen vorliegt – mit Erdalkalimetallen wie Ca, Mg und Erdmetallen, wie B, Tl, einen Ionenaustausch vornimmt oder dass man die Zeolithe mit Lösungen von Salzen der genannten Metalle imprägniert.

Eine Dotierung der Zeolithe mit Übergangsmetallen, wie W, Fe, Zn, mit Edelmetallen wie Pd und mit seltenen Erdmetallen wie Ce, La ist vorteilhaft.

Einen Ionenaustausch nimmt man z.B. vor, indem man den verformten Pentasilzeolithen in einem Steigrohr vorlegt und bei 20 bis 100° C z.B. eine wässerige Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Beim Imprägnieren wird das zeolithische Material z.B. mit einem Halogenid, einem Nitrat oder einem Oxid der voranbeschriebenen Metalle in wässeriger oder alkoholischer Lösung behandelt. Sowohl an einen Ionenaustausch als auch an eine Imprägnierung schliesst sich zumindest eine Trocknung, wahlweise eine abermalige Calcination an.

Im einzelnen kann man z.B. so verfahren, dass man Wolframsäure $H_2WO_4$ oder $Ce(NO_3)_3 \cdot 6\,H_2O$ in Wasser – grösstenteils zumindest – löst und mit dieser Lösung den verstrangten oder unverstrangten Zeolithen eine gewisse Zeit, ca. 30 min, tränkt. Das Restwasser wird am Rotationsverdampfer abgezogen. Dann wird der getränkte Zeolith bei ca. 150° C getrocknet und bei ca. 550° C calciniert. Dieser Tränkvorgang kann mehrmals hintereinander vorgenommen werden, um den gewünschten Metallgehalt einzustellen. Man kann auch z.B. eine ammoniakalische $Pd(NO_3)_2$-Lösung herstellen und darin den reinen pulverförmigen Zeolithen bei 40 bis 100° C unter Rühren ca. 25 h aufschlämmen. Nach Abfiltrieren, Trocknen bei ca. 150° C und Calcination bei ca. 500° C kann das so gewonnene zeolithische Material mit und ohne Bindemittel zu Strängen oder Pellets weiterverarbeitet werden. Ein Ionenaustausch des in der H-Form vorliegenden Zeolithen kann so vorgenommen werden, dass man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80° C im Kreislauf 15 bis 20 h leitet. Danach wird mit Wasser ausgewaschen, bei ca. 150° C getrocknet und bei ca. 550° C calciniert.

Als Katalysatoren kommen auch amorphe Aluminosilikate und/oder Aluminophosphate in Betracht.

Amorphe Aluminosilikate sind Verbindungen aus $Al_2O_3$ und $SiO_2$. Derartige Verbindungen lassen sich beispielsweise aus $Al_2(SO_4)_3 \cdot 18\,H_2O$ und Wasserglas herstellen. Dabei verfährt man z.B. so, dass man $Al_2(SO_4)_3 \cdot 18\,H_2O$ und Wasserglas bei pH 1 bis 3, bevorzugt bei pH 1, in Gegenwart von $H_2SO_4$ zusammengibt. Danach wird diese Mischung mit einer Ammoniak-Lösung bei pH 4 bis 8, bevorzugt 6, bei 0 bis 50° C, bevorzugt 20 bis 30° C umgesetzt.

Der hierbei entstandene Niederschlag wird abfiltriert, mit Ammoniumcarbonat-Lösung gewaschen und bei 50° C bis 150° C getrocknet. Besonders vorteilhafte Katalysatoren werden z.B. erhalten, wenn man ein $SiO_2$:$Al_2O_3$-Verhältnis von 80:20 bis 20:80, insbesondere 75:25 bis 35:65 einstellt.

Aluminophosphate können beispielsweise durch Fällung aus $Al(NO_3)_3 \cdot 9\,H_2O$ mit $(NH_4)_3PO_4$ oder $(NH_4)_2HPO_4$ oder $(NH_4)H_2PO_4$ bei unterschiedlichen pH-Werten, insbesondere bei pH 3 bis pH 10, synthetisiert werden. Nach Abfiltrieren und Waschen wird das erhaltene Produkt bei 50 bis 150° C, vorzugsweise 60 bis 100° C, getrocknet und bei 300° C bis 900° C, vorzugsweise 500 bis 800° C, calciniert.

Die so hergestellten Aluminosilikate und Aluminophosphate können in reiner Form ohne Bindemittel zu Strängen oder Tabletten oder Wirbelgut verarbeitet und eingesetzt werden. Man kann sie aber auch nach Isolierung, Trocknung und eventueller Calcination mit verschiedenen Bindern zu Strängen oder Tabletten verformen. Das gewichtsmässige Verhältnis Silikat bzw. Phosphat zu Bindemittel beträgt 90:10 bis 30:70. Als Bindemittel eignen sich diverse Aluminiumoxide, bevorzugt Boehmit, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge bei 110° C/16 h getrocknet und bei 500° C/16 h calciniert.

Zur Erhöhung der Selektivität, der Standzeit und der Anzahl an Regenerierungen kann man diese Katalysatoren auch modifizieren. Eine Modifizierung besteht z.B. darin, dass man den unverformten oder verformten Katalysator mit Alkalimetallen wie Na – sofern nicht schon von der Synthese her vorhanden – mit Erdalkali wie Ca, Mg und Erdmetallen wie B, Tl dotiert. Insbesondere die Dotierung der Aluminosilikate und -phosphate mit Übergangsmetallen wie W, Fe, Zn mit Edelmetallen wie Pd und mit seltenen Erdmetallen wie Ce, La ist vorteilhaft. Bei manchen metalldotierten Katalysatoren ist eine Nachbehandlung mit Wasserstoff vorteilhaft.

Eine weitere Möglichkeit der Modifizierung besteht darin, dass man die Zeolithe, die Aluminosilikate oder Aluminophosphate – verformt oder unverformt – einer Behandlung mit Säuren wie Salzsäure, Flusssäure und Phosphorsäure und/oder mit Wasserdampf unterwirft. Auch kann durch partielle Verkokung (pre-coke) die Aktivität des

Katalysators für ein Selektivitätsoptimum des gewünschten Reaktionsproduktes eingestellt werden.

Nach einer Desaktivierung der Katalysatoren, die beim Verfahren der Erfindung durch Koksabscheidung eintreten kann, lassen sich diese durch Abbrennen der Koksablagerung mit Luft oder mit einem Luft/$N_2$-Gemisch bei 400 bis 550° C, bevorzugt 500° C, in einfacher Weise regenerieren, wodurch sie ihre Anfangsaktivität zurückerhalten. Die Katalysatoren können wahlweise als 2 bis 4 mm Stränge oder als Tabletten mit 3 bis 5 mm Durchmesser oder als Pulver mit Teilchengrössen von 0,1 bis 0,5 mm oder als Wirbelgut eingesetzt werden.

*Beispiele 1 bis 11*

2,5-Diethyl-2-formyl-2,3-dihydro-4H-pyran wurde zum Zwecke der Spaltung in Tiglinaldehyd und Ethylacrolein unter isothermen Bedingungen in einen Rohrreaktor (Wendelform, 0,6 cm Innendurchmesser, 90 cm Länge) eingeführt und in der Gasphase bei 400° C über den Katalysator geleitet. Die Versuchsdauer betrug 6 Stunden. Die Reaktionsprodukte wurden destillativ abgetrennt und durch Siedepunkt, Brechungsindex und NMR-Spektren charakterisiert. Die quantitative Bestimmung der Reaktionsprodukte I und III und der Ausgangsprodukte II erfolgte gaschromatographisch. Die Art des Katalysators, die Belastung (WHSV), der Umsatz und die Selektivität sind der folgenden Tabelle zu entnehmen.

*Beispiel 11* (Vergleichsbeispiel)

In diesem Falle wurden in den Reaktor anstelle des Katalysators Quarzkörner als Wärmeträger gefüllt. Ansonsten wurde wie angegeben verfahren.

*Tabelle*

| Beispiel | 1 | 2 | 3 | 4 | 5 | 6 | 7 | 8 | 9 | 10 | 11 |
|---|---|---|---|---|---|---|---|---|---|---|---|
| Katalysator | A | B | C | D | E | F | G | H | I | J | – |
| Temperatur (°C) | 400 | 400 | 400 | 400 | 400 | 400 | 400 | 400 | 400 | 400 | 400 |
| WHSV | 2 $h^{-1}$ | 2 $h^{-1}$ | 2 $h^{-1}$ | 2 $h^{-1}$ | 2,3 $h^{-1}$ | 2,4 $h^{-1}$ | 2 $h^{-1}$ | 2 $h^{-1}$ | 2 $h^{-1}$ | 2,3 $h^{-1}$ | 2,3 $h^{-1}$ |
| Umsatz (%) | 98,9 | 98,4 | 98,9 | 100 | 96,7 | 99,6 | 99 | 100 | 99,0 | 100 | 100 |
| Selektivität Tiglinaldehyd (%) | 10,7 | 31,2 | 27,0 | 9,9 | 8,5 | 15,0 | 24,5 | 7,7 | 16,9 | 13,0 | – |
| Selektivität Ethylacrolein (%) | 77,2 | 52,2 | 54,7 | 79,6 | 72,8 | 72,9 | 59,6 | 84,4 | 68,7 | 77,0 | 92,3 |

Die verwendeten Katalysatoren wurden folgendermassen hergestellt:

*Katalysator A*

In einem Rührautoklaven wurden 65 g hochdisperses $SiO_2$ und 20,3 g $Al_2(SO_4)_3 \cdot 18\ H_2O$ in 1 kg einer wässerigen 1,6-Hexandiamin-Lösung (Mischung 50:50 Gew.%) bei autogenem Druck auf 150° C erhitzt. Nach Abfiltrieren und Auswaschen wurde das kristalline Reaktionsprodukt bei 110° C/24 h getrocknet und bei 500° C/24 h calciniert. Man erhielt einen Aluminosilikatzeolith vom Pentasil-Typ, der 91,6 Gew.% $SiO_2$ und 4,6 Gew.% $Al_2O_3$ enthielt. Dieses Produkt wurde mit Boehmit im Gewichtsverhältnis 60:40 zu 2 mm-Strängen verformt und bei 110° C/16 h getrocknet und bei 500° C/24 h calciniert.

*Katalysator B*

In einem Rührautoklaven wurden 64 g hochdisperses $SiO_2$, 12,2 g $H_3BO_3$, 800 g einer wässerigen 1,6-Hexandiamin-Lösung (Mischung 50:50 Gew.%) unter autogenem Druck auf 170° C erhitzt. Nach Abfiltrieren und Auswaschen wurde das kristalline Reaktionsprodukt bei 100° C/24 h getrocknet und bei 500° C/24 h calciniert. Der so erhaltene Borosilikatzeolith setzte sich zusammen aus 94,2 Gew.% $SiO_2$ und 2,32 Gew.% $B_2O_3$. Mit diesem Material wurden durch Verformen mit Boehmit im Gewichtsverhältnis 60:40 2 mm-Stränge hergestellt, die bei 110° C/16 h getrocknet und bei 500° C/24 h calciniert wurden.

*Katalysator C*

In einem Rührautoklaven wurde bei autogenem Druck ein Gemisch aus 273 g Wasserglas, gelöst in 253 g einer wässerigen 1,6-Hexandiamin-Lösung (Mischung 50:50 Gew.%) und 31 g Eisensulfat, gelöst in 21 g 96%iger Schwefelsäure und 425 g Wasser 4 Tage auf 165° C erhitzt. Danach wurde der so erhaltene Eisensilikatzeolith abfiltriert, ausgewaschen, bei 100° C/24 h getrocknet und bei 500° C/24 h calciniert. Er hatte ein $SiO_2/Fe_2O_3$-Verhältnis von 17,7 und einen $Na_2O$-Gehalt von 0,62 Gew.%. Dieser Eisensilikatzeolith von Pentasil-Typ wurde mit Boehmit im Gewichtsverhältnis 60:40 zu 2 mm-Strängen verstrangt, bei 110° C/16 h getrocknet und bei 500° C/24 h calciniert.

*Katalysator D*

Ein NaY-Zeolith, der mit Boehmit im Verhältnis 60:40 verstrangt ist, wurde zum Zwecke des Ionenaustausches bei 80° C mit einer Lösung von 15 g $NH_4Cl$ in 85 g Wasser behandelt und dann bei 500° C calciniert. Der Ammoniumaustausch mit anschliessender Calcination wurde so oft wiederholt, bis der Rest-Natriumgehalt 0,21 Gew.% betrug.

*Katalysator E*

Der unter der Bezeichnung Zeolon 900® (Norton Chemical Process Products, USA) im Handel erhältliche Zeolith vom H-Mordenit-Typ wurde mit Siliciumdioxid im Verhältnis 60:40 zu 2 mm-Strängen verformt.

*Katalysator F*

Katalysator B wurde mit einer wässerigen $H_2WO_4$-Lösung ca. 30 min getränkt. Dann wurde das Wasser der überstehenden Restlösung am Rotationsverdampfer abgezogen. Danach wurde der Katalysator bei 130° C getrocknet und bei 550° C calciniert. Der Vorgang wurde wiederholt, bis der Katalysator einen W-Gehalt von 4 Gew.% aufwies.

*Katalysator G*

Es wurde wie bei der Herstellung von Katalysator F verfahren, wobei jedoch eine wässerige Lösung von $Ce(NO_3)_3 \cdot 6\ H_2O$ verwendet wurde. Die Tränkung wurde auf einen Ce-Gehalt des Katalysators von 7,2 Gew.% eingestellt.

*Katalysator H*

Über Katalysator B wurde eine ammoniakalische $Pd(NH_3)_4(NO_3)_2$-Lösung im Kreis gepumpt (65 ml/min). Danach wurde bei 110° C getrocknet und bei 500° C calciniert. Der Pd-Gehalt betrug 3,3 Gew.%.

*Katalysator I*

Beim Katalysator B wurde durch Behandlung mit einer 20%igen NaCl-Lösung bei 80° C ein Ionenaustausch vorgenommen. Nach der Calcination bei 500° C enthielt der Katalysator 0,28 Gew.-% Na.

*Katalysator J*

589 g $Al_2(SO_4)_3 \cdot 18\ H_2O$ wurden mit 989 g Wasserglas (27,3 Gew.% $SiO_2$-Gehalt) in Gegenwart von $H_2SO_4$ bei pH = 1,5 zusammengegeben. Das Gemisch wurde mit 1 l $H_2O$ verdünnt. Die so erhaltene Lösung und 677 g Ammoniak-Lösung wurden im Zeitraum von 25 min unter Rühren und bei einem pH von 6 parallel in eine Vorlage mit 2 l $H_2O$ gegeben. Nach 1 h Rühren wurde der gebildete Niederschlag abfiltriert und mit 0,5 gew.%iger $(NH_4)_2CO_3$-Lösung gut ausgewaschen. Es wurde ein Aluminosilikat mit einem $SiO_2$:$Al_2O_3$-Verhältnis von 75:25 erhalten. Das Aluminosilikat wurde tablettiert und bei 110° C/16 h getrocknet.

## Patentansprüche

1. Verfahren zur Herstellung von 2-Methyl-2-alkenalen der Formel

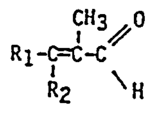 I,

in der $R_1$ und $R_2$ Wasserstoffatome, Alkylreste mit 1 bis 18 Kohlenstoffatomen oder aromatische Reste bedeuten, *dadurch gekennzeichnet*, dass man Dihydropyrane der Formel

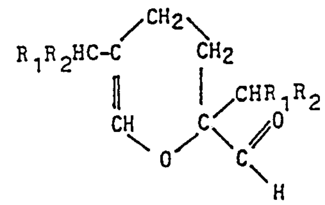 II,

in der $R_1$ und $R_2$ die obengenannte Bedeutung haben, in Gegenwart von Zeolithen, amorphen Aluminosilikaten und/oder Aluminophosphaten erhitzt und aus den dabei entstehenden Gemischen die 2-Methyl-2-alkenale abtrennt.

2. Verfahren nach Anspruch 1, *dadurch gekennzeichnet*, dass man als Dihydropyran 2,5-Diethyl-2-formyl-2,3-dihydro-4H-pyran verwendet.

3. Verfahren nach Anspruch 1, *dadurch gekennzeichnet*, dass man einen Zeolith des Pentasil-Typs verwendet.

4. Verfahren nach Anspruch 1, *dadurch gekennzeichnet*, dass man einen Aluminosilikat-Zeolith verwendet.

5. Verfahren nach Anspruch 1, *dadurch gekennzeichnet*, dass man einen Borosilikatzeolith verwendet.

6. Verfahren nach Anspruch 1, *dadurch gekennzeichnet*, dass man einen Eisensilikatzeolith verwendet.

7. Verfahren nach Anspruch 1, *dadurch gekennzeichnet*, dass man einen Aluminiumsilikatzeolith des Y-Typs verwendet.

8. Verfahren nach Anspruch 1, *dadurch gekennzeichnet*, dass man einen Aluminiumsilikatzeolith mit Mordenit-Struktur verwendet.

9. Verfahren nach Anspruch 1, *dadurch gekennzeichnet*, dass man ein amorphes Aluminosilikat mit einem gewichtsmässigen $SiO_2$:$Al_2O_3$-Verhältnis von 80:20 bis 20:80 verwendet.

10. Verfahren nach Anspruch 1, *dadurch gekennzeichnet*, dass man ein Aluminophosphat verwendet, das bei einem pH-Wert von 3 bis 10 gefällt wurde.

## Claims

1. A process for the preparation of a 2-methylalken-2-al of the formula

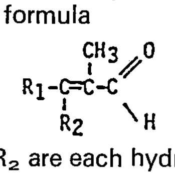 I,

where $R_1$ and $R_2$ are each hydrogen, alkyl of 1 to 18 carbon atoms or an aromatic radical, wherein a dihydropyran of the formula

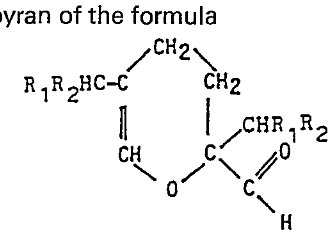 II,

where $R_1$ and $R_2$ have the above meanings, is heated in the presence of a zeolite, an amorphous aluminosilicate and/or an aluminophosphate, and the 2-methylalken-2-al is separated from the mixture formed.

2. A process as claimed in claim 1, wherein 2,5-diethyl-2-formyl-2,3-dihydro-4H-pyran is used as the dihydropyran.

3. A process as claimed in claim 1, wherein a zeolite of the pentasil type is used.

4. A process as claimed in claim 1, wherein an aluminosilicate zeolite is used.

5. A process as claimed in claim 1, wherein a borosilicate zeolite is used.

6. A process as claimed in claim 1, wherein a ferrosilicate zeolite is used.

7. A process as claimed in claim 1, wherein a Y-type aluminosilicate zeolite is used.

8. A process as claimed in claim 1, wherein an aluminosilicate zeolite having the structure of mordenite is used.

9. A process as claimed in claim 1, wherein an amorphous aluminosilicate is used in which the weight ratio of $SiO_2$ to $Al_2O_3$ is from 80:20 to 20:80.

10. A process as claimed in claim 1, wherein an aluminophosphate is used which has been precipitated at a pH of 3 to 10.

## Revendications

1. Procédé de préparation de 2-méthyl-2-alcénals de la formule

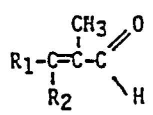

I

dans laquelle R[1] et R[2] représentent des atomes d'hydrogène, des radicaux alkyle comportant de 1 à 18 atomes de carbone ou des radicaux aromatiques, caractérisé en ce que l'on chauffe des dihydropyrannes de la formule

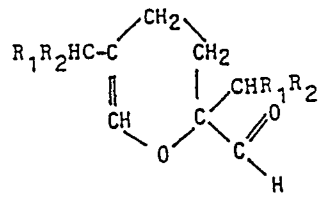

II

dans laquelle R[1] et R[2] possèdent les significations qui leur ont été attribuées ci-dessus, en présence de zéolithes, d'aluminophosphates et/ou d'aluminosilicates amorphes et en ce que l'on sépare ensuite les 2-méthyl-2-alcénals des mélanges ainsi formés.

2. Procédé suivant la revendication 1, caractérisé en ce que l'on utilise le 2,5-diéthyl-2-formyl-2,3-dihydro-4H-pyranne à titre de dihydropyranne.

3. Procédé suivant la revendication 1, caractérisé en ce que l'on utilise une zéolithe du type pentasil.

4. Procédé suivant la revendication 1, caractérisé en ce que l'on utilise une zéolithe du type aluminosilicate.

5. Procédé suivant la revendication 1, caractérisé en ce que l'on utilise une zéolithe du type borosilicate.

6. Procédé suivant la revendication 1, caractérisé en ce que l'on utilise une zéolithe du type ferrisilicate.

7. Procédé suivant la revendication 1, caractérisé en ce que l'on utilise une zéolithe du type aluminosilicate du genre Y.

8. Procédé suivant la revendication 1, caractérisé en ce que l'on utilise une zéolithe du type aluminosilicate à structure de mordénite.

9. Procédé suivant la revendication 1, caractérisé en ce que l'on utilise un aluminosilicate amorphe qui possède un rapport pondéral $SiO_2:Al_2O_3$ de 80:20 à 20:80.

10. Procédé suivant la revendication 1, caractérisé en ce que l'on utilise un aluminophosphate précipité à une valeur de pH de 3 à 10.